# EUROPEAN PATENT SPECIFICATION

(11) **EP 1 741 462 B1**
(45) Date of publication and mention of the grant of the patent: **14.11.2007**
(21) Application number: 05425480.0
(22) Date of filing: 06.07.2005
(51) Int. Cl.: A61M 16/08, H05B 3/56

(54) **Flexible duct with heating devices and elements for transmitting parameters sensed by sensing probes**
Flexibler Schlauch mit Heizelementen und Elementen zur Übertragung von sensorisch erfassten Parametern
Conduit flexible avec dispositifs de chauffage, muni d'éléments pour transmettre des paramètres détectés avec des sondes de mesure

(43) Date of publication of application: 10.01.2007
(73) Proprietor: Deas S.R.L., 48014 Castel Bolognese RA (IT)
(72) Inventor: Scardovi, Domenico, 48014 Castel Bolognese (Prov.of Ravenna) (IT)
(74) Representative: Modiano, Micaela Nadia

(56) References cited:
- DE-A1- 19 647 548
- US-A- 4 621 633
- US-A- 4 686 354
- US-A- 5 357 948
- US-B1- 6 190 480
- US-B1- 6 219 490

## Description

The present invention relates to a flexible duct with devices for heating the conveyed stream, provided with elements for transmitting the parameters sensed by means of sensing probes connected thereto.

Respiration ducts or tubes that administer air to a patient must have a constant internal passage section and must therefore be non-deformable, because any compression thereof would entail a reduction of the stream of air sent to the patient, altering the ventilation parameters suitable to ensure adequate ventilation.

These ducts can be connected to suitable humidifiers/heaters, which are adapted to inject warm and humidified air into the tubular body in order to make it reach the patient.

In order to prevent the forming of condensation inside the ducts due to the decreases of the temperature of the dispensed streams during passage, it has been thought to distribute, along the outer surface of the tubular body, appropriate heating devices suitable to keep the surface warm; the air, in passing, flows over these surfaces and is warmed.

These drawbacks have been obviated by distributing, on the outer surface of suitable tubular bodies, stiffening crests and suitable heating devices, which keep the surface warm.

The streams dispensed to the patient must be monitored so that they comply with specific physical and chemical parameters (usually temperature, humidity, carbon dioxide, concentrations of anesthetic, and other volatile substances).

The practice consists in introducing probes at an end portion (or at both end portions) of the duct: the probes are connected to the unit that controls them and manages the data acquired by means of external cables, which can be independent or coupled to the duct itself.

The presence of these cables is potentially dangerous, since they may become caught (if they are independent), causing dangerous tractions on the end portion to which they are connected, with the consequent risk of disconnection; in any case, they increase the weight of the duct and make it more awkward to handle for the patient even when the cables are locked on the surface of the duct.

Moreover, since the monitoring lines are meant for multiple use, they are reused several times on different patients and can be the cause of cross-infections.

US-A-4 686 354 discloses a composite flexible delivery hose for use with a medical humidifier that comprises a combination of elements as set forth in the pre-characterizing portion of the appended claim 1.

The aim of the present invention is to obviate the cited shortcomings and meet the mentioned requirements, i.e., to provide a flexible duct with devices for heating the conveyed stream which is provided with elements for transmitting the parameters sensed by means of sensing probes connected thereto, in which the cables for the connection and power supply of the probes are not bulky and do not protrude from the duct.

Within this aim, an object of the present invention is to provide a single-use duct in order to improve hygiene and avoid cross-contamination of patients.

Another object of the present invention is to provide a duct that has a modest cost.

A further object of the present invention is to provide the duct with a structure that is simple, relatively easy to provide in practice, safe in use, and effective in operation.

In accordance with the invention, there is provided a flexible duct with devices for heating the conveyed stream, as defined in the appended claims.

Further characteristics and advantages of the invention will become better apparent and evident from the detailed description that follows of a preferred but not exclusive embodiment of a flexible duct with devices for heating the conveyed stream, provided with elements for transmitting the parameters sensed by means of sensing probes connected thereto, illustrated by way of non-limiting example in the accompanying drawings, wherein:

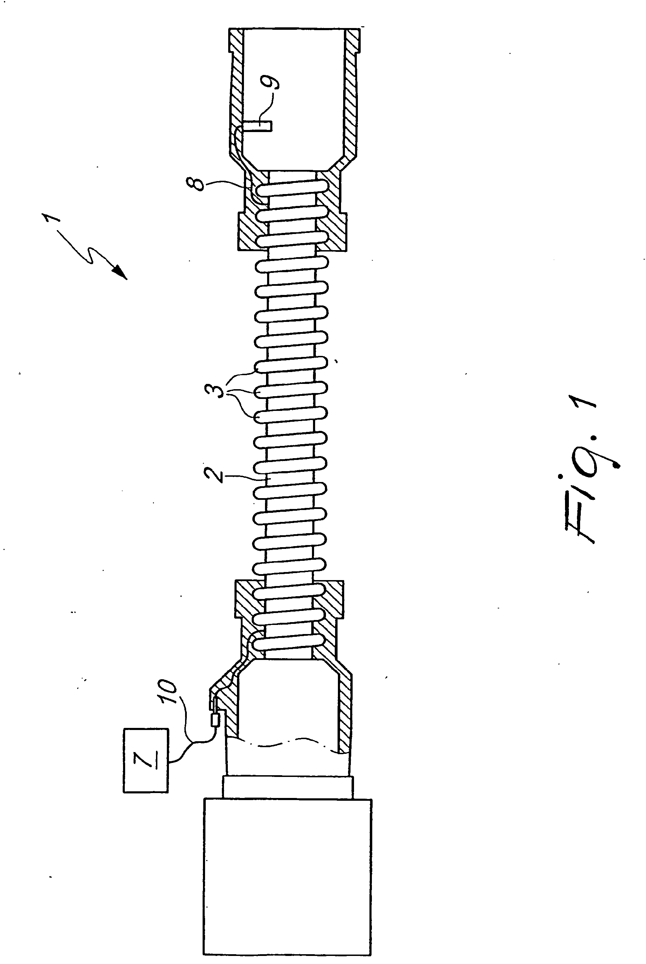

Figure 1 is a schematic side view of a possible embodiment of a flexible duct according to the invention, installed on the respective supply and control assemblies;
Figure 2 is a schematic partial side view of a portion of the first possible embodiment of a flexible duct according to the invention;
Figure 3 is a partially schematic side view of a portion of a second possible embodiment of a flexible duct according to the invention;
Figure 4 is a partially schematic side view of a portion of a third possible embodiment of a flexible duct according to the invention;
Figure 5 is a partially schematic side view of a portion of a fourth possible embodiment of a flexible duct according to the invention.

With reference to the figures, the reference numeral 1 generally designates a flexible duct provided with devices for heating the conveyed stream and with verification probes.

The duct 1 comprises a flexible tubular body 2, on the outer surface of which a wirelike stiffening element 3, made of highly rigid polymeric material, and at least one heating filament 4 (constituted for example, in a particular embodiment, by a metallic conductor through which electric current flows, heating it by virtue of the Joule effect) are distributed substantially in a helical configuration. The filament 4 is generally covered by a respective sheath and/or by a layer 5 of insulating thermoplastic material.

At least one cable 6 is also provided on the outer surface of the body 2 and is substantially distributed in a helical configuration; the cable 6 is adapted to transmit a signal according to the protocol of the management and control unit 7 that is associated with it.

The cable 6 has a first end 8, which is connected electrically to at least one respective probe 9, and a second end 10, which is connected to the unit 7: in this manner, the unit 7 can store information regarding physical and chemical characteristics of the air stream that reaches the patient. For diagnostic and therapeutic purposes, it can be important for medical staff and for the patient to know exactly the temperature (also in order to control it and keep it within the required standards) or the humidity or concentration of certain volatile substances or other parameters that can contribute to alleviate the problems of the patient.

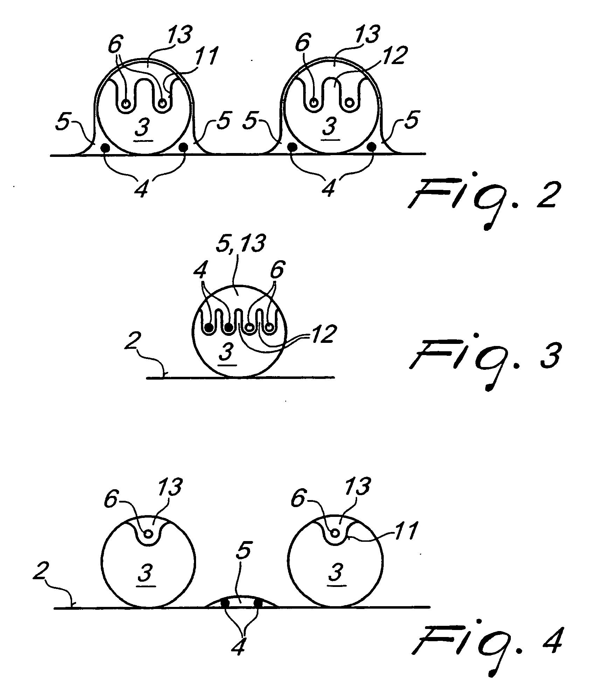

In one constructive solution that is of particular practical interest (some possible variations of which are shown in Figures 2, 3 and 4), the wirelike element 3 is provided with at least one longitudinal groove 11 for accommodating the cable 6 (or a plurality of cables 6).

The longitudinal grooves 11 can in fact be multiple and mutually separated by a crest 12 arranged radially with respect to said duct 1; in this manner, each groove 11 is suitable to contain a respective cable 6, keeping it separate from the others.

It should also be noted that the longitudinal grooves 11 can also be provided in such a number as to contain a respective heating filament 4: in this case, the cables 6 can be arranged along the outer surface of the tubular body 2.

Advantageously, as shown in Figure 3, it is also possible to provide on the element 3 a number of longitudinal grooves 11 such as to contain multiple cables 6 and multiple heating filaments 4, with the advantage of grouping all the electrical components in a single portion of the duct 1.

In any case, it should be noted that the grooves 11, which accommodate respective cables 6 and/or stems 4, have an external covering 13 made of polymeric material, which is suitable to prevent unintentional contact with them (insulation): if the cables 6 and the stems 4 are both present, it can be noted that the insulating layer 5 and the covering 13 coincide in a single block of polymeric material.

From a functional standpoint, it is very important that the external covering 13 (or the layer 5) be easily removable from the groove 11: the covering 13 (or the layer 5) is therefore made of a highly deformable material with respect to the element 3 on which the groove 11 is formed, in order to allow easy separation thereof from the walls of the groove 11 and facilitate the operations that prepare for wiring, such as the extraction of a conductor and the removal of the insulation.

It has thus been shown that the invention achieves the proposed aim and objects.

The invention thus conceived is susceptible of numerous modifications and variations, all of which are within the scope of the appended claims.

All the details may furthermore be replaced with other technically equivalent ones.

In the illustrated embodiments, individual characteristics, given in relation to specific examples, may actually be interchanged with other different characteristics that exist in other embodiments.

Moreover, it is noted that anything found to be already known during the patenting process is understood not to be claimed and to be the subject of a disclaimer.

In practice, the materials used, as well as the shapes and the dimensions, may be any according to the requirements without thereby abandoning the protective scope of the claims that follow.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A flexible duct with devices for heating the conveyed stream, provided with elements for transmitting the parameters sensed by means of sensing probes connected thereto, the duct comprising a flexible tubular body (2), on the outer surface of which a wirelike stiffening element (3) and at least one heating filament (4) are distributed substantially in a helical arrangement; at least one cable (6), which is distributed on the outer surface of said tubular body (2) substantially along a helix and has a first end (8) connected electrically to at least one respective probe (9) of said sensing probes and a second end (10) connected to a control and management unit (7), **characterized in that** said wirelike element (3) is provided with at least one longitudinal groove (11) for accommodating said at least one cable (6) and/or said at least one heating filament (4).

2. The duct according to claim 1, **characterized in that** it comprises a plurality of longitudinal grooves (11) that are mutually separated by a crest (12) arranged radially with respect to said duct (1), each one of said grooves (11) being adapted to contain a respective cable (6).

3. The duct according to claim 1, **characterized in that** it comprises a plurality of longitudinal grooves (11) that are mutually separated by a crest (12), which is arranged radially with respect to said duct (1), each one of said grooves (11) being adapted to contain a respective heating filament (4).

4. The duct according to claims 2 and 3, **characterized in that** said longitudinal grooves (11) are in such a number as to contain multiple cables (6) and multiple heating filaments (4).

5. The duct according to one or more of the preceding claims 2-4, **characterized in that** said longitudinal grooves (11), which accommodate respective cables (6) and for heating filaments (4), are provided with an outer covering (5, 13) made of polymeric material, which is adapted to prevent unintentional contact with them.

6. The duct according to claim 5, **characterized in that** said outer covering (13) can be removed from said longitudinal grooves (11) being made of a material that is highly deformable with respect to the element (3) on which the groove (11) is formed.

## Patentansprüche

1. Eine flexible Rohrleitung mit Vorrichtungen zum Erhitzen des übermittelten Stromes, bereitgestellt mit Elementen zum Übertragen der mit Hilfe von daran verbundenen Meßfühlern ermittelten Parameter, die Rohrleitung umfassend, einen flexiblen röhrenförmigen Körper (2) auf dessen Außenfläche ein drahtähnliches Versteifungselement (3) und mindestens ein Heizfaden (4) in einer im Wesentlichen schraubenartigen Anordnung verteilt sind; mindestens ein Kabel (6), das auf der Außenfläche des röhrenförmigen Körpers (2) im Wesentlichen entlang einer Spirale verteilt ist und ein erstes Ende (8) aufweist, das elektrisch mit mindestens einem jeweiligen Messfühler (9) verbunden ist und ein zweites Ende (10), das mit einer Steuer- und Betriebseinheit (7) verbunden ist, **dadurch gekennzeichnet, dass** das drahtähnliche Element (3) mit mindestens einer Längsnut (11) zur Aufnahme des mindestens einen Kabels (6) und/oder des mindestens einen Heizdrahtes (4) bereitgestellt ist.

2. Die Rohrleitung gemäß Anspruch 1, **dadurch gekennzeichnet, dass** sie eine Mehrzahl von Längsnuten (11) umfasst, die gegenseitig durch einen Kamm (12) getrennt sind, der in Bezug auf die Rohrleitung (1) radial angeordnet ist, wobei jede der Nuten (11) angepasst ist, um ein jeweiliges Kabel (6) zu beinhalten.

3. Die Rohrleitung gemäß Anspruch 1, **dadurch gekennzeichnet, dass** sie eine Mehrzahl von Längsnuten (11) umfasst, die gegenseitig durch einen Kamm (12) getrennt sind, der in Bezug auf die Rohrleitung (1) radial angeordnet ist, wobei jede der Nuten (11) angepasst ist, um einen jeweiligen Heizdraht (4) zu beinhalten.

4. Die Rohrleitung gemäß den Ansprüchen 2 und 3, **dadurch gekennzeichnet, dass** die Längsnuten (11) in solch einer Anzahl vorhanden sind, um mehrere Kabel (6) und mehrere Heizdrähte (4) zu beinhalten.

5. Die Rohrleitung gemäß einem oder mehreren der vorhergehenden Ansprüche 2-4, **dadurch gekennzeichnet, dass** die Längsnuten (11), die jeweilige Kabel (6) und/oder Heizdrähte (4) aufnehmen, mit einer aus Polymermaterial hergestellten äußeren Abdeckung (5, 13) bereitgestellt sind, die angepasst ist, um unbeabsichtigten Kontakt mit ihnen zu verhindern.

6. Der Rohrleitung gemäß Anspruch 5, **dadurch gekennzeichnet, dass** die äußere Abdeckung (13) aus den Längsnuten (11) entfernt werden kann, da es aus einem Material hergestellt ist, das in Bezug auf das Element (3) auf der die Nut (11) ausgebildet ist, hoch verformbar ist.

## Revendications

1. Conduit flexible avec dispositifs de chauffage du courant convoyé, muni d'éléments pour la transmission de paramètres détectés au moyen de sondes de mesure connectées à celui-ci, le conduit comprenant un corps tubulaire flexible (2), sur la surface externe duquel un élément de raidissement filiforme (3) et au moins un filament de chauffage (4) sont distribués sensiblement selon un agencement en hélice; au moins un câble (6), qui est distribué sur la surface externe dudit corps tubulaire (2) sensiblement le long d'une hélice et qui présente une première extrémité (8) connectée électriquement à au moins une sonde respective (9) desdites sondes de mesure et une deuxième extrémité (10) connectée à une unité (7) de commande et de gestion, **caractérisé en ce que** ledit élément filiforme (3) est muni d'au moins une rainure longitudinale (11) pour loger ledit au moins un câble (6) et/ou ledit au moins un filament de chauffage (4).

2. Conduit selon la revendication 1, **caractérisé en ce qu'**il comprend une pluralité de rainures longitudinales (11) qui sont mutuellement séparées par une crête (12) agencée radialement par rapport audit conduit (1), chacune desdites rainures (11) étant adaptée pour contenir un câble respectif (6).

3. Conduit selon la revendication 1, **caractérisé en ce qu'**il comprend une pluralité de rainures longitudinales (11) qui sont mutuellement séparées par une crête (12) agencée radialement par rapport audit conduit (1), chacune desdites rainures (11) étant adaptée pour contenir un filament de chauffage (4) respectif.

4. Conduit selon les revendications 2 et 3, **caractérisé en ce que** lesdites rainures longitudinales (11) sont d'un nombre tel à pouvoir contenir des câbles multiples (6) et des filaments de chauffage multiples (4).

5. Conduit selon une ou plusieurs des revendications précédentes 2 à 4, **caractérisé en ce que** lesdites rainures longitudinales (11), qui logent des câbles (6) et/ou des filaments de chauffage (4) respectifs, sont munis d'une couverture externe (5, 13) réalisée en un matériau polymérique, qui est adapté pour empêcher un contact involontaire avec eux.

6. Conduit selon la revendication 5, **caractérisé en ce que** la couverture externe (13) peut être enlevée desdites rainures longitudinales (11) étant réalisée en un matériau qui est hautement déformable par rapport à l'élément (3) sur lequel la rainure (11) est formée.
